(19) Europäisches Patentamt
European Patent Office
Office européen des brevets

(11) EP 2 157 434 A1

(12) EUROPEAN PATENT APPLICATION

(43) Date of publication:
24.02.2010 Bulletin 2010/08

(51) Int Cl.:
$G01N\ 33/74^{(2006.01)}$  $G01N\ 33/92^{(2006.01)}$
$B01D\ 59/44^{(2006.01)}$  $C07C\ 1/00^{(2006.01)}$
$G01N\ 1/00^{(2006.01)}$

(21) Application number: 08252710.2

(22) Date of filing: 15.08.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicants:
• Imperial Innovations Limited
London SW7 2AZ (GB)
• The University of Nottingham
Nottingham NG7 2RD (GB)

(72) Inventors:
• Sephton, Mark A.
London, SW7 2AX (GB)

• Snape, Colin E.
Nottingham, NG7 2RD (GB)
• Gomes, Rachel L.
London, SW7 2AX (GB)
• Meredith, Will
Nottingham, NG7 2RD (GB)

(74) Representative: Hedley, Nicholas James Matthew et al
Kilburn & Strode LLP
20 Red Lion Street
London
WC1R 4PJ (GB)

(54) **Preparation of biological samples for compound-specific carbon isotope analysis**

(57) The present invention provides a method of measuring the relative ratios of $^{13}C$ to $^{12}C$ in an organic compound, e.g. a steroid or a conjugated steroid such as the sulphate conjugate of testosterone, using compound-specific carbon isotope analysis. The method comprises:

subjecting a sample of the compound to hydropyrolysis in the presence of a transition metal catalyst, preferably platinum but also including palladium, rhodium and iridium, under a hydrogen atmosphere at a temperature below 350°C,

subjecting the hydrocarbon product released via hydropyrolysis to gas chromatography to provide a purified sample of the hydropyrolysed product,
combusting the hydropyrolysed product to form carbon dioxide and submitting the resulting carbon dioxide to mass spectrometry to obtain the relative ratios of $^{13}C$ to $^{12}C$ in the carbon dioxide.

The method deconjugates the compound from both sulfate or glucuronide moieties when the compound is derived from urine samples and avoids the need to derivatise the compound prior to the gas chromatography step.

Figure 2. GC//MS traces of the hydropyrolysis products for the free steroids, testosterone and 5α-androstane-3α, 17β-diol and their conjugates, testosterone-17-sulfate and 5α-androstane-3α, 17β-diol-17-glucuronide.

**Description**

Technical Field

The present invention relates to a method of preparing a biological sample for compound-specific carbon isotope analysis.

Background art

The testing of urine samples for the presence of physiological compounds, for example steroids, is well-established. Such physiological compounds may be conjugated with glucuronide or sulfate groups, which are added by the body to render them more soluble in urine so that they can be excreted; this is particularly the case with poorly-soluble compounds such as steroids.

One particularly prominent area where such testing techniques are used is to detect the presence of drugs that enhance performance or productivity and one category of such drugs is formed by those that occur naturally in the body but are artificially supplemented. Examples of such drugs include testosterone, epitestosterone, androsterone, etiocholanolone and DHEA (dehydroepiandosterone). These materials are excreted from the body in urine in a form that is conjugated with a glucuronide or a sulfate group.

$^{13}C$ and $^{12}C$ are naturally occurring stable isotope of carbon. Steroids destined for pharmaceutical applications are produced by modifying steroid compounds from plants and the ratio of $^{13}C$ to $^{12}C$ in these sources is lower than in humans, which provides a useful route to ascertain whether a steroid found in a urine sample is naturally occurring or whether it is the result of artificial administration. This may be achieved by compound-specific carbon isotope analysis, which involves combusting the steroid of interest and measuring the $^{13}C$ / $^{12}C$ ratio in the resulting carbon dioxide using mass spectrometry and comparing the measured ratio for the compound of interest with the corresponding ratio of a naturally occurring steroid from the same test subject that does not enhance performance and is unaffected by the administration, e.g. cholesterol. If the ratios are not similar that is indicative of the test subject having received artificial steroid supplements. However, it is necessary to isolate the steroids of interest before they can be tested.

There are obviously established methods of isolating organic materials from mixtures. One of these used for compound-specific carbon isotope analysis is to isolate a crude sample using, for example, solid phase extraction followed by high performance liquid chromatography (HPLC). The compound of interest may then be separated out by gas chromatography. However, in the case of compounds that are conjugated with glucuronide and/or sulfate groups, the glucuronide and sulfate groups prevent the separation of the steroid via gas chromatography since they pass inefficiently through the gas chromatography column. Therefore, in order to render them suitable for isolation by gas chromatography, the analyte compounds must be deconjugated, e.g. enzymatically or by chemical hydrolysis, to remove the glucuronide and sulfate groups. They then have to be derivatized by the addition of a small group, e.g. by silyation or acylation, to improve their volatility, thermostability and peak shape and so make them suitable for separation by gas chromatography.

Both deconjugation and derivatization give rise to further problems. Enzymatic hydrolysis can only be used to cleave the glucuronide conjugate but is highly inefficient against sulfated conjugates, while chemical hydrolysis can cleave both groups but can cause analyte degradation.

Derivatization using silyation or acylation has disadvantages. Acylation introduces carbon atoms into the test compound and accordingly gives rise to uncertainty in the test measurement of the $^{13}C/^{12}C$ isotope ratio of the compound. Silyation does not introduce any carbon atoms but the measurement of the isotope ratios involve combusting the steroid to form carbon dioxide and the silyation causes deposition of silicon on the metal wires used in the combustion chamber, which can lead to incomplete combustion and therefore inaccurate measurement. There is a need for a procedure to prepare samples for the gas chromatography step in compound-specific carbon isotope analysis that is simpler and more reliable than the deconjugation and derivitization steps discussed above.

In an article by Sephton et al, Rapid Commun. Mass. Spectrom. 2005; 19:323-325, there is reported a method for preparing samples for the compound-specific carbon isotope analysis of fatty acids, which involves hydropyrolysing the sample prior to gas chromatography, which defunctionalises the fatty acids of the fatty acid carboxyl moieties but retains their carbon skeleton intact in the form of the corresponding n-alkanes. As reported, the hydropyrolysis involves the catalytic addition of hydrogen to the carbon skeleton in the presence of a molybdenum catalyst. In particular, the sample is adsorbed onto silica before adding a dispersed molybdenum catalyst precursor $((NH_4)_2MoO_2S_2)$ from solution using standard incipient wetness procedures. During hydropyrolysis, the molybdenum compound decomposes *in situ* above 250°C to form a catalytically-active molybdenum sulfide phase (see Analytical Chemistry Insights, 2007; 2, 37-42). The hydropyrolysis itself typically takes place by heating the sample bed in the reactor resistively from ambient to 250°C at a rate of 250°C min$^{-1}$, and then from 250°C up to 520°C at 8°C min$^{-1}$ under a hydrogen pressure of 15 MPa. This additional hydropyrolysis step was found to provide much sharper peaks in the subsequent carbon isotope analysis than the corresponding fatty acid, which provides better resolution when analysing a complex mixture. It is also reported that the hydropyrolysis can be achieved with a very high yield (95%) and the article in Analytical Chemistry Insights, 2007; 2, 37-42 suggests that this technique provides effective determination of the carbon isotopic composition of individual fatty acids without the use of derivatizing agents.

In a further article by Sephton et al in Rapid Communications in Mass. Spectrometry 19; 3339-3342, it is reported that the same hydropyrolysis technique can be applied to steroids, in particular 5β-cholanic acid, 5a-cholestanol and cholesterol and that the hydropyrolysis delicately strips the functional groups from fatty acids and steroids, retaining the carbon skeleton and stereochemistry and that hydropyrolysis has the potential to eliminate the current derivatization steps and enable gas chromatography-combustion-isotope ratio mass spectrometry (GC-C-IRMS) analysis of any steroid. Unfortunately it was found that the cholesterol rearranged during the hydropyrolysis procedure to yield after defunctionalization the two expected cholestane isomers, four diasteranes, and an unresolved complex mixture comprising other diasteranes, cholestenes and diasterenes. Multiple products are less suitable for effective carbon isotope analysis because one cannot be sure that the $^{13}$C will not preferentially report to the rearranged (or the unrearranged) hydrocarbons and so the isotope ratios measured for the individual rearranged and the unrearranged by compound-specific carbon isotope analysis may be different and thus reduce the overall reliability of the procedure.

The above article by Sephton et al in Analytical Chemistry Insights, which is a review article, concludes:

"a catalyst system and a temperature regime that minimises such rearrangement should be a high priority for future development".

An additional objective is to solve the problems surrounding the necessary steroid deconjugation step prior to their analysis by GC-C-IRMS. No current universal (glucuronide and sulfate) deconjugation technique exists. There is no published work on the use of the hydropyrolysis technique as a deconjugation procedure.

The present invention provides a method of preparing samples for compound-specific carbon isotope analysis.

Disclosure of the Invention

The process of the present invention is defined in the accompanying claims.

Broadly stated, the present invention provides a method of preparing biological samples for compound-specific carbon isotope analysis. It has been found that, by using a platinum, or other suitable Group VIII transition metals including palladium, rhodium and iridium, it is possible to provide complete hydropyrolysis of biological samples at much lower temperatures than have previously been achieved, thereby reducing the extent of rearrangement that can occur during the defunctionalization of the hydrocarbon skeletons. The transition metal catalyst may form from below 1 to over 20% of the total content of the supported catalyst.

The hydropyrolysis protocol in accordance with the present invention avoids the need to derivatize the biological sample for separation using gas chromatography since hydropyrolysis defunctionalizes the biological sample, i.e. removes the functional groups from the compound leaving the original hydrocarbon chain or the original cyclic skeleton of the parent compound, thereby removing any functional groups that could interfere with the gas chromatography separation step. In the case of conjugated compounds derived from urine, the procedure also removes glucuronide and sulfate conjugate moieties.

The catalyst, e.g. platinum, is preferably supported on activated carbon but other supports can be used, including silica, alumina, titiania and zirconia. The lower temperature employed allows the reaction product to remain adsorbed on the adsorbent in the reactor, from where they can subsequently be recovered for analysis. The temperature of the hydropyrolysis is preferably maintained at not greater than 400°C, for example, not greater than 350°C, e.g. 325°C or below. In an example described in detail below, the hydropyrolysis at a temperature of 300°C leads to the complete cleavage of conjugate groups attached to steroid compounds in addition to the loss of all other functional groups. In addition, the products of hydropyrolysis are shown to have the same ratio of $^{13}$C /$^{12}$C as the parent steroid, thereby indicating that there is no fractionation of the carbon isotopes by the hydropyrolysis step.

In addition to detecting the carbon isotopic ratio in steroids, the same techniques can be used for other physiological compounds, especially compounds with a hydrocarbon skeleton augmented by heteroatom-containing functional groups e.g. various acids, alcohols and ketones. The hydrocarbon skeletons include those in lipids, fatty acids, steroids and metabolised polycyclic aromatic hydrocarbons. The physiological compounds can be obtained from a variety of sources, for example, urine, swab samples, blood samples and other biological samples.

The techniques find application not only in detecting excess steroid levels in sports men and women but also in research and diagnosis to track the progress through the body of various compounds by enriching them or a precursor with $^{13}$C. The use of $^{13}$C has substantial advantages over the labelling of compounds with the non-stable, or radioactive, isotope $^{14}$C. Owing to its radioactivity, $^{14}$C can lead to detrimental physiological effects, which are particularly unacceptable when dealing with paediatric patients. In contrast, compounds enriched with the stable isotope $^{13}$C have no negative

physiological effect.

Other applications are found in the forensic field where post-mortem detection of externally and involuntarily administered drugs, such as gamma-hydroxybutyric acid, can be distinguished from naturally occurring counterparts. Sources and transport/distribution networks of drugs (geographical origin and tracking of individual batches) can also be ascertained using this method.

Moreover, authentication and adulteration of foodstuffs (e.g. fatty acids in olive oils and the detection of illegal steroid use in meat production) enables protection of the food chain. Environmental contamination from industrial or social practices (e.g. the release of endocrine disrupters) is also another application.

Brief Description of Drawings

There will now be described, by way of example only, a method in accordance with the present invention, which will be described by reference to the accompanying drawings in which:

Figure 1 shows the chemical structures of (a) 5a androstane-3a,17β diol and (b) its glucuronide conjugate and (c) testosterone and (d) its sulfate conjugate; and

Figure 2 shows the mass spectrometer traces of the hydropyrolysis products of the steroids and their conjugates shown in Figure 1.

Example 1

Steroids can be isolated from a urine samples using known techniques, which essentially involves a two-step isolation procedure, the first step comprising a solid phase extraction step using a solid phase binder that selectively binds to the steroid. Following elution from the solid phase, the steroid is subject to the second separation step using high performance liquid chromatography (HPLC), which provides either the individual steroid or a simple mixture comprising the steroid.

The steroid obtained from the extraction and separation steps is subject to hydropyrolysis, as more fully described in Example 2, and then to gas chromatography (GC) to separate the hydropyrolysis products. The isolated product of interest is combusted (C) in the presence of oxygen to form carbon dioxide, which is analysed using an isotope ratio mass spectrometer (IRMS), which is a specialised mass spectrometer in which mass spectrometric methods are used to measure the relative abundance of isotopes in a given sample. The ratio of $^{13}C$ relative to $^{12}C$ is measured relative to a standard and the results are expressed in the form:

$$d\ ^{13}C = [[^{13}C\ /\ ^{12}C]_{sample}\ /\ [^{13}C\ /\ ^{12}C]_{standard} - 1]\ x\ 1,000$$

to provide the amount of $C^{13}$ relative to $C^{12}$, expressed in parts per 1,000 (symbol ‰).

Example 2

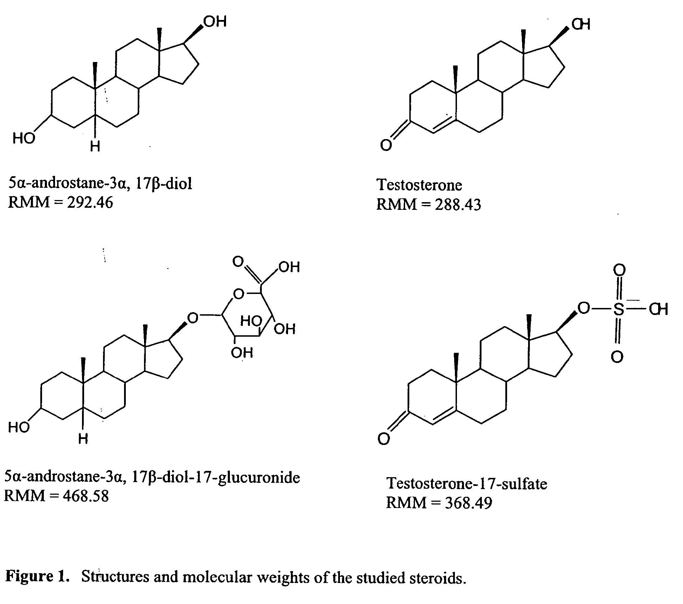

The free steroids 5a- androstane-3a,17β diol and testosterone and their conjugates 5a- androstane-3a, 17β diol-17 glucuronide and testosterone- 17 sulfate shown in Figure 1 were obtained from Makaira Limited (London, UK). The standard used in the calculation of d $^{13}C$ was 5β cholane, which was obtained from the hydropyrolysis of 5β cholanic acid as described in a paper by Meredith et al, Org. Geochem. 2006; 37;1705.

For each analyte, aliquots of the steroids were adsorbed on a bed formed of 200 mg of activated carbon on which was supported 5 wt % platinum. The steroids were subjected to hydropyrolysis at 300°C and 15 MPa of hydrogen as described in the paper by Sephton et al in Rapid Commun. Mass. Spectrom. 2005; 19:323. Briefly, this procedure involves heating the samples resistively from 50°C to 100°C at 300°C per minute and then from 100°C to 300°C at 4°C per minute, under a hydrogen pressure of 15 MPa. To enable complete defunctionalization, an additional step of holding the reaction at 300°C for 8 minutes was included. A hydrogen sweep gas flow of 5 litres per minute, measured at ambient temperature and pressure, ensured that gaseous products were quickly removed from the reactor vessel. In contrast to the procedure described in the above article, the low temperatures employed ensured that the defunctionalized products, together with any unreacted material, remained adsorbed on the activated carbon in the reactor rather than being swept by the hydrogen flow into a dry ice cool silica trap. The products and the unreacted material were then recovered from the activated carbon with dichloromethane and methanol for subsequent analysis.

A GC-C-IRMS (gas chromatography-combustion-isotope ratio mass spectrometry) unit (Delta+XP), (Thermo Finnigan) was used for the determination of compound-specific $^{13}C/^{12}C$ isotope ratios of the hydropyrolysis products of the free and conjugated steroids (the tests were performed in triplicate). Separation of the pyrolysis products via gas chromatography was performed on a silica capillary column (DB5, 30 m x 0.25 $\mu$m) with helium as the carrier gas and a temperature program of 180°C (hold for 2 minutes) to 300°C (hold for 5 minutes) at 8°C per minute.

The carbon isotopic composition of 5a-androstane-3a,17β diol and testosterone are set out in Table 1, both compounds being measured both as free steroids and as conjugates using combustion (C)-IRMS (the measurements were taken from the samples supplied from the source specified above and did not involve hydropyrolysis). They were also subjected to hydropyrolysis to enable isotopic measurements using GC-C-IRMS. The errors marked in Table 1 are given at 1 s.

**Table 1.**

| Compound Type | d$^{13}$ (‰) | | Technique |
| --- | --- | --- | --- |
| | Testosterone | 5a-androstane-3a,17β diol | |
| Free steroid | -28.2 ($\pm$0.0) | -30.6 ($\pm$0.1) | C-IRMS |
| Hydropyrolysis product of free steroid | -28.4 ($\pm$0.3) | -30.7($\pm$0.2) | GC-C-IRMS |
| Conjugated steroid | -28.1 ($\pm$0.0) | -26.2 ($\pm$0.3) | C-IRMS |
| Hydropyrolysis product of conjugated steroid | -28.2 ($\pm$0.3) | -31.4 ($\pm$0.2) | GC-C-IRMS |

For hydropyrolysis to be used as a preparative technique in place of the current deconjugation and derivatization steps of conjugated steroids, it is necessary to show that the structures of both the conjugate and the free parent steroids are similar following hydropyrolysis and that there is no associated isotopic fractionation i.e. preferential reporting of one or other isotope as a result of hydropyrolysis.

The results in Table 1 for free steroids 5a- androstane-3a,17β diol and testosterone and the hydropyrolysis products of the free steroids illustrate that the isotopic composition of the hydropyrolysis product is consistent with the starting material. No isotopic fractionation is evident for unconjugated steroids that pass through the hydropyrolysis procedure.

The sulfated testosterone conjugate provides an opportunity to assess any carbon isotopic fractionation brought about by the deconjugation step. As the sulfate moiety contains no carbon, its isotopic composition can be measured directly by C-IRMS (as supplied without pyrolysis) and compared to the corresponding hydropyrolysis product by GC-C-IRMS. Any difference in the isotopic ratios observed following hydropyrolysis must be result of kinetic isotope effects associated with the hydropyrolysis step. From Table 1, it is evident that the hydropyrolysis products of testosterone sulfate are faithful expressions of the starting testosterone sulfate with no isotopic conversion resulting from the conversion of the conjugated steroids to the deconjugated hydrocarbon skeleton.

In contrast to the sulfated conjugate, the 5a-androstane-3a, 17β diol glucuronide conjugate has additional carbon atoms in the glucuronide moiety. It has therefore different isotope ratio values for the conjugate and deconjugated forms. In biological systems, carbohydrates often exhibit $^{13}$C enrichment relative to other components and it is therefore no surprise that the glucuronide conjugate appears isotopically heavier than the deconjugated steroid following loss of the glucuronide sugar group.

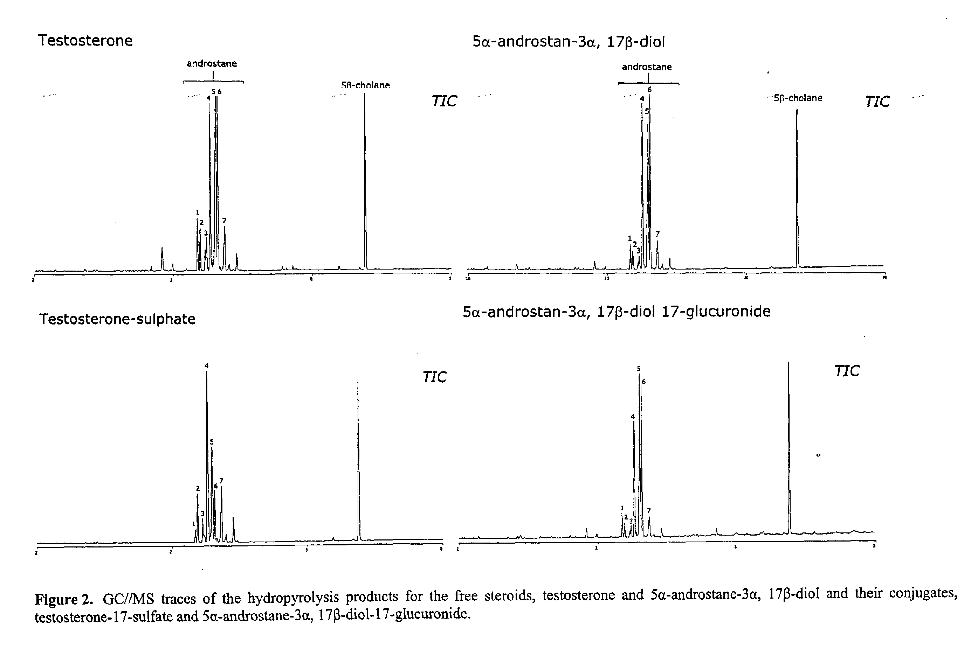

Figure 2 shows that the hydropyrolysis products for the two conjugates appear to be structurally representative of their respective parent free steroid. This reveals that the hydropyrolysis procedure effectively removes the conjugate moiety and any functional groups attached to the steroid skeleton to leave the carbon skeleton (including the carbon chain derived from any functional groups attached to the skeleton). The defunctionalization of the conjugate occurs irrespective of whether it is a glucuronide or a sulfate moiety.

The use of the supported platinum catalyst in place of the molybdenum catalyst used in the article by Sephton et al Rapid Commun. Mass. Spectrom. 2005; 19:3339 results in less rearrangement and superior peak shapes.

## Claims

**1.** A method of measuring the relative ratios of $^{13}$C to $^{12}$C in an organic compound using compound-specific carbon isotope analysis, which method comprises:

subjecting a sample of the compound to hydropyrolysis in the presence of a transition metal catalyst, preferably platinum but also including palladium, rhodium and iridium, under a hydrogen atmosphere at a temperature below 350°C,

subjecting the hydrocarbon product released via hydropyrolysis to gas chromatography to provide a purified sample of the hydropyrolysed product,
combusting the hydropyrolysed product to form carbon dioxide and
submitting the resulting carbon dioxide to mass spectrometry to obtain the relative ratios of $^{13}C$ to $^{12}C$ in the carbon dioxide.

**2.** A method as claimed in claim 1, wherein the compound that is submitted to hydropyrolysis is part of a mixture of compounds.

**3.** A method as claimed in claims 1 or 2, wherein the compound has a hydrocarbon skeleton augmented by heteroatom-containing functional groups including acids, alcohols and ketones.

**4.** A method as claimed in claims 3, wherein the hydrocarbon skeleton is one present in lipids, fatty acids, steroids and metabolised polycyclic aromatic hydrocarbons.

**5.** A method as claimed in any preceding claim, wherein the compound is conjugated with a sulfate or glucuronide moiety.

**6.** A method as claimed in claim 5, which includes the step of isolating the conjugated compound from urine.

**7.** A method as claimed in any preceding claim, wherein the hydropyrolysis reaction is conducted in the presence of a material that adsorbs the hydropyrolysis products at the reaction temperature and the method further comprises desorbing the hydropyrolysis products following the completion of the hydropyrolysis step.

**7.** A method as claimed in claim 6, wherein the adsorbent is selected from one of the following classes: activated carbon, silica, titania, alumina and zirconia.

**8.** A method as claimed in claim 6, wherein the transitional metal catalyst, preferably platinum, is supported on the adsorbent.

**9.** A method as claimed in any preceding claims wherein the catalyst is a supported catalyst and the total content of the catalyst in the supported catalyst is from 1 to 20% by weight.

**10.** A method as claimed in any preceding claim, wherein the catalyst is a Group VIII transition metal, preferably platinum but also including palladium, rhodium and iridium.

**11.** A method as claimed in any preceding claim, wherein the catalyst is in elemental (metallic) form.

5α-androstane-3α, 17β-diol
RMM = 292.46

Testosterone
RMM = 288.43

5α-androstane-3α, 17β-diol-17-glucuronide
RMM = 468.58

Testosterone-17-sulfate
RMM = 368.49

**Figure 1.** Structures and molecular weights of the studied steroids.

**Figure 2.** GC//MS traces of the hydropyrolysis products for the free steroids, testosterone and 5α-androstane-3α, 17β-diol and their conjugates, testosterone-17-sulfate and 5α-androstane-3α, 17β-diol-17-glucuronide.

EP 2 157 434 A1

Europäisches
Patentamt

European
Patent Office

Office européen
des brevets

**EUROPEAN SEARCH REPORT**

Application Number

EP 08 25 2710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | SEPHTON M A ET AL: "Biomedical and forensic applications of combined catalytic hydrogenation-stable isotope ratio analysis" ANAL CHEM INSIGHTS, vol. 2, 2007, pages 37-42, XP002510341 * the whole document * in particular par. spanning p. 38 and p. 39; p. 40, col. 2, l. 9 to p. 42, col. 1, last line. ----- | 1-11 | INV. G01N33/74 G01N33/92 B01D59/44 C07C1/00 G01N1/00 |
| D,A | MEREDITH ET AL: "The use of model compounds to investigate the release of covalently bound biomarkers via hydropyrolysis" ORG GEOCHEM, vol. 37, no. 12, 8 December 2006 (2006-12-08), pages 1705-1714, XP005797200 * the whole document * in particular par. 3.3 on p. 1712. ----- | 1-11 | |
| | | | TECHNICAL FIELDS SEARCHED (IPC) G01N B01D C07C |

The present search report has been drawn up for all claims

| Place of search | Date of completion of the search | Examiner |
|---|---|---|
| Munich | 15 January 2009 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 157 434 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Sephton et al.** *Rapid Commun. Mass. Spectrom.,* 2005, vol. 19, 323-325
- *Analytical Chemistry Insights,* 2007, vol. 2, 37-42
- **Sephton et al.** *Rapid Communications in Mass. Spectrometry,* vol. 19, 3339-3342
- **Meredith et al.** *Org. Geochem.,* 2006, vol. 37, 1705
- *Rapid Commun. Mass. Spectrom.,* 2005, vol. 19, 323
- **Sephton et al.** *Rapid Commun. Mass. Spectrom.,* vol. 19, 3339